# EUROPÄISCHE PATENTSCHRIFT

(11) **EP 1 811 040 B1**
(45) Veröffentlichungstag und Bekanntmachung des Hinweises auf die Patenterteilung: **07.01.2009**
(21) Anmeldenummer: 06124265.7
(22) Anmeldetag: 17.11.2006
(51) Int. Cl.: C12Q 1/68, G01N 33/543, G01N 33/558, G01N 33/559

(54) **Vorrichtung und Verfahren zum Nachweis eines Analyten**
Device and method for the detection of an analyte
Dispositif et procédé pour détecter un analyte

(30) Priorität: 23.01.2006 DE 102006003177
(43) Veröffentlichungstag der Anmeldung: 25.07.2007
(73) Patentinhaber: SIEMENS AKTIENGESELLSCHAFT, 80333 München (DE)
(72) Erfinder: Bangert, Joachim, Dr., 91052 Erlangen (DE); Bär, Ludwig, Dr., 91056 Erlangen (DE); Ehben, Thomas, 91085 Weisendorf (DE); Feucht, Hans-Dieter, Dr., 71272 Renningen (DE); Zilch, Christian, Dr., 04275 Leipzig (DE)

(56) Entgegenhaltungen:
- WO-A-03/019188
- WO-A-03/043931
- WO-A-20/06104700
- DE-A1- 19 503 664

## Beschreibung

Die Erfindung betrifft eine Vorrichtung und ein Verfahren zum Nachweis und/oder zur Quantifizierung eines Analyten mithilfe von Fängermolekülen, die spezifisch an den Analyten binden. Derartige Vorrichtungen bzw. Verfahren werden im Stand der Technik insbesondere zur Detektion vom Biomolekülen wie Nukleinsäuren oder Proteinen verwendet. Im Falle von Protein-Assays koppeln die Proteine z.B. mit spezifischen, monoklonalen Antikörpern, während zur Detektion von DNA- oder RNA-Strängen jeweils komplementäre Nukleinsäuren als Fängermoleküle verwendet werden.

Die WO-A 2006/104700 offenbart einen magnetischen Proteinsensor zur Detektion eines Analyten, mit einem magnetischen Partikel, der an einem stabförmigen Protein gebunden ist, an das eine Bindungsgruppe angeordnet ist, die spezifisch den Analyten unter Bildung eines Sensor-Analyt-Komplexes bindet. Eine hierdurch hervorgerufene Änderung von Größe und Form des Partikels wird durch Vergleich mit der magnetischen Suszeptibilität oder der Relaxationszeit des Sensors ohne Analyten festgestellt. Hierzu ist ein externes Wechselmagnetfeld notwendig, das gemäß einem Messverfahren an eine flüssige Probe angelegt wird, die vorher mit dem Proteinsensor versetzt wurde.

Die WO-A 2003/019188 offenbart ein auf gleichem Prinzip beruhendes Verfahren und Vorrichtung zur Detektion der Relaxation von magnetischen Partikeln, die mit dem Analyten über ein am Partikel befestigtes Molekel verbunden sind. Hierbei wird die Frequenz des externen Wechselmagnetfelds so gewählt, dass der Imaginärteil der magnetischen Suszeptibilität möglichst gering ist.

Hierin zitiert ist die DE-A 195 03 664, welche ein Verfahren zur quantitativen Detektion von Analyten in Fest- und Flüssigphasen offenbart. Hierbei wird die sich durch spezifische Bindung des Analyten an einem magnetischen Teilchen verändernde Relaxation über die Zeit ausgelesen, wobei vorher die Teilchen magnetisiert werden.

Die WO-A 2003/043931 offenbart magnetische Partikel mit speziellen Oberflächeneigenschaften, die durch daran immobilisierte Gruppen bereitgestellt sind. Die Anbindung eines Analyten wird hierbei mittels Veränderungen der Beweglichkeit beobachtet.

Die meisten Nukleinsäure- und Protein-Detektionsverfahren mittels Biosensoren bedürfen einer Markierung der zu untersuchenden Biomoleküle. Bei den hierfür verwendeten molekularen Markern kann es sich um fluoreszierende, lumineszierende oder elektrisch oder magnetisch aktive Moleküle oder Partikel (Quantum-Dots, magnetische Beads) handeln. Nach einer Hybridisierung z.B. mit komplementären DNA-Fängermolekülen in homogener Phase oder an einem Mikroarray werden die Marker an den gebundenen Biomolekülen mittels optischer, elektrischer oder magnetischer Messverfahren detektiert.

Eine Ausnahme stellt das gravimetrische Verfahren dar, das mit Hilfe von Piezosensoren die Masseveränderung durch Anlagerung von Molekülen an die Sensoroberfläche detektiert. Hier ist keine Markierung der Nukleinsäuren oder Proteine erforderlich. Nachteilig erweist sich aber die relativ niedrige Sensitivität, der geringe dynamische Messbereich und das relativ aufwändige Produktionsverfahren zur Herstellung der Piezoschwinger unter den Sensorschichten.

Bei den bekannten Array-basierten Detektionsverfahren werden vor der Hybridisierung oder Bindung des Analyten an die Fängermoleküle Marker an die nachzuweisenden Probenmoleküle (Analyten) gebunden. Diese Marker werden nach einer erfolgten Hybridisierung oder Bindung detektiert. Dazu wird das Mikroarray in eine Auswerteeinheit eingebracht, die die optischen, elektrischen oder magnetischen Markersignale erfasst. Die Auswerteinheit enthält zu diesem Zweck ein Sensor-Array, das die Signalemission jedes einzelnen Array-Punktes parallel oder sequentiell misst. Hierbei ergeben sich hohe Anforderungen an Integration und Homogenität des Sensor-Arrays.

Es ist Aufgabe der vorliegenden Erfindung einen höheren Integrationsgrad für die magnetische Detektion und/oder Quantifizierung von Analyten bereit zustellen.

Diese Aufgabe wird mit den Merkmalen des kennzeichnenden Teils des Anspruchs 1 gelöst. Durch Anordnen der magnetisierten Nanopartikel in mehreren Schichten kann ein hoch integriertes Detektions-Array bereit gestellt werden. Zum anderen trägt ein solches "Multilayer" Design zur Erzeugung eines Gesamtmagnetfeldes bei, wodurch zudem die Empfindlichkeit zum Nachweis des Analyten erheblich gesteigert werden kann.

Hierzu wird eine Vorrichtung vorgeschlagen, welche eine Grundmatrix aufweist, und mehrere magnetisierte Nanopartikel in der Grundmatrix beweglich angeordnet sind und an denen die Fängermoleküle verankert sind, welche spezifisch an einen Analyten binden, wobei die Bewegbarkeit der Nanopartikel in der Grundmatrix durch eine Bindung des nachzuweisenden Analyten an die Fängermoleküle beeinflussbar ist und die veränderte Bewegbarkeit der Nanopartikel magnetisch auslesbar ist. Mit "binden" ist nicht nur eine kovalente Bindung gemeint, sondern z.B. auch eine Hybridisierung.

Erfindungsgemäß ist die Grundmatrix eine Gelmatrix. Eine vernetzte Gelmatrix kann beispielsweise auf Polyacrylamid, Polyacrylsäure, Polyhydroxyethylmethacrylat, Polyvinylalkohol oder Polyvinylpyrrolidon oder auf Copolymeren basieren, die entsprechende Monomersequenzen enthalten. Die Vernetzung der Gelmatrix kann zum Beispiel thermisch oder durch UV-Licht erfolgen, bevor oder nachdem die Nanopartikel eingebracht wurden.

Die Nanopartikel, im Folgenden auch "Beads" (von Engl. "Kügelchen") genannt, haben vorzugsweise eine zumindest annäherungsweise sphärische oder elliptische Form und einen Durchmesser von ca. 10nm-10µm, insbesondere 20nm-1µm und besonders bevorzugt ca. 50-500nm. Sie können z.B. gemäß dem in dem Artikel von Albrecht M. et al. "Magnetic multilayers on nanopsheres", nature materials, 2005, Seiten 1-4 beschriebenen Verfahren hergestellt sein, also einen Kern aus Polystyrol und eine magnetische Beschichtung, insbesondere aus CoPb, aufweisen.

Bevorzugt sind die Beads jedoch anders aufgebaut, z.B. umfassen sie gemäß einer besonders bevorzugten Ausführungsform eine Kugel aus Polystyrol, die mit magnetischen Partikeln durchsetzt ist. Der Grundstoff Polystyrol ermöglicht eine gute Anbindung der Fängermoleküle an der Oberfläche. Die magnetischen Partikel weisen vorzugsweise eine längliche, insbesondere nadelförmige, Form auf, so dass diese sich innerhalb des Polystyrols nicht drehen können und somit die Magnetisierungsrichtung des Beads festgelegt ist.

Alternativ können die Beads massiv aus einem magnetischen Material aufgebaut sein. Das magnetische Material der Beads bzw. der in diesen enthaltenen magnetischen Partikel kann beispielsweise CoPb, CoPt, CrO₂, CrFe, Ni-Verbindungen oder NdFeB sein.

Die Beads sollten lediglich eine Magnetisierung, insbesondere eine Dipol-Magnetisierung, aufweisen. Die Beads haben vorzugsweise anisotrope, hysteretische Eigenschaften, d.h. sie behalten über einen definierten magnetischen Feldstärkebereich ihre Magnetisierungsachse in Bezug auf ihre Polarachse bei. Die Hysterese ihrer magnetischen Charakteristik bewirkt, dass die Nanopartikel bei den unten beschriebenen Schreibvorgängen nicht ummagnetisiert werden, sondern lediglich ihre räumliche Orientierung ändern, etwa wie eine Kompassnadel. Das Bead ist so beschaffen, dass eine Anisotropieachse für eine feste Orientierung der magnetischen Polarisation relativ zur Bead-Geometrie sorgt.

Die Fängermoleküle können auf beliebige Weise an den Nanopartikeln verankert sein, insbesondere können sie als Beschichtung auf die Oberfläche der Nanopartikel aufgebracht sein. Ggf. können sie auch über Linker-Moleküle mit dem Nanopartikel verbunden sein.

Bevorzugt werden eine Anzahl benachbarter Beads jeweils zu eine Gruppe, im Folgenden "Spot" zusammengefasst. Diese sind vorzugsweise jeweils mit identischen Fängermolekülen ausgestattet. Vorzugsweise sind viele Spots auf einer Grundmatrix in Form einer Matrix oder eines "Arrays" angeordnet, z.B. in einem 5x5 bis 50x50 Array. Der "Pitch", d.h. der Mittenabstand der Spots, beträgt z.B. 1-500µm, vorzugsweise 10-100µm. Der Durchmesser eines einzelnen Spots beträgt z.B. 0,5 bis 1 Pitch. Bei einem Durchmesser von 1 Pitch berühren sich benachbarte Spots, was prinzipiell möglich ist. Die Grundfläche der einzelnen Spots ist z.B. kreisförmig oder rechteckig mit abgerundeten Ecken.

Die Vernetzung der Gelmatrix wird vorzugsweise durch Steuerung der Vernetzungsdichte derart ausgestaltet, dass die magnetischen Beads einerseits dauerhaft in der Grundmatrix eingeschlossen sind, andererseits jedoch noch ganz oder teilweise gedreht werden können, wodurch auch die Anisotropieachse des Bead-Magnetfeldes (Position von magnetischem Nord- und Südpol) verändert wird. Haben alle Beads eines Spots die gleiche magnetische Orientierung, geht vom Spot ein Magnetfeld aus, dessen Orientierung der der einzelnen Beads entspricht. Mit einem Magnetfeldsensor ist es möglich, diese Orientierung messtechnisch zu erfassen. Wird dieser Sensor ähnlich wie bei einer herkömmlichen Speicherfestplatte als Schreib-Lesekopf ausgeführt, lässt sich damit zusätzlich die Magnetfeldorientierung eines Spots ändern. Bei einer solchen Ummagnetisierung eines Spots werden alle zum Spot gehörenden Beads gedreht.

Bevorzugt ist der nachzuweisende Analyt ein Biomolekül, z.B. eine Nukleinsäure oder ein Protein, und die Fängermoleküle Nukleinsäuren oder monoklonale Antikörper, wobei der Analyt durch Hybridisierung bzw. Antigen-Antikörper Reaktion an die Fängermoleküle bindet.

Um mit einem Array solcher Beads in einem Gel Biomoleküle in einer Probe nachzuweisen, werden diese nachzuweisenden Biomoleküle vorzugsweise in einem oder mehreren Aufbereitungsschritten aus der Probe isoliert und anschließend auf das Gel aufgetragen. Sie gelangen zur Oberfläche der Beads. Dies kann entweder durch Diffusion durch das Gel erfolgen oder dadurch, dass die Beads an der Geloberfläche angeordnet sind und dadurch aus diesem teilweise herausragen. Die An- oder Abwesenheit von Biomolekülen verändert nun die mechanischen und damit die magnetischen Eigenschaften des Arrays. Dies kann auf dreierlei Weisen geschehen:

Gemäß einer ersten Variante binden die Zielmoleküle spezifisch an Fängermoleküle auf dem Bead. Allein hierdurch wird die Drehbarkeit der Beads in der Gelmatrix ganz oder teilweise unterbunden, insbesondere für den Fall, dass es sich um große Moleküle, z.B. lange Molekülketten wie lange DNA- oder RNA-Stränge oder Aminosäureketten, oder um sterisch anspruchsvolle Moleküle handelt. Die Drehbarkeit der Beads wird z.B. durch die molekulare Verschlingung der Zielmoleküle mit der umgebenden Matrix verringert. Die Hybridisierung oder Bindung an die Beadoberfläche kann auch außerhalb der Matrix an deren Grenzfläche stattfinden. Hierbei ist eine Diffusion der Zielmoleküle in die Matrix hinein nicht erforderlich. Der Grad der Einschränkung der Drehbarkeit wird hierbei durch die Anzahl der Fänger-Zielmolekül-Komplexe pro Bead bestimmt. Für diese Detektionsvariante ist es ausreichend, dass die Fängermoleküle ggf. nur eine spezifische Sequenz der nachzuweisenden DNA- oder RNA-Stränge adressieren.

Gemäß einer zweiten Variante sind die Beads eines Spots jeweils so beschichtet, dass die Zielmoleküle Molekülbrücken zwischen den Beads ausbilden können. Bevorzugt sind hierfür die Beads in einem Spot mit mindestens zwei verschiedenen Fängermolekülen ausgestattet, die gegenüber unterschiedlichen Bereichen des nachzuweisenden Zielmoleküls spezifisch sind. Beispielsweise werden hierfür spezifische Zielsequenzen am Beginn und Ende eines gesuchten Moleküls ausgewählt. Durch die Molekülbrücken zwischen den Beads kommt es zu einer deutlichen Einschränkung der Beweglichkeit der magnetischen Beads, durch die sie an einer freien Drehung gehindert werden bzw. nach einer geringen Drehung durch ein externes Magnetfeld wieder in ihre Ausgangsposition zurückdrehen. Das Vorhandensein nachzuweisender Moleküle kann also dadurch erfasst werden, dass die Beads sich in einem Magnetfeld nicht ausrichten. D.h. es kann eine "Magnetisierung" eines Spots in einer anschließenden Messung nicht nachgewiesen werden. Der Spot verhält sich also ohne Hybridisierung oder Bindung wie ein beschreibbares Bit einer Festplatte und mit Hybridisierung oder Bindung wie ein nicht beschreibbares Bit. Bei dieser Variante ist der Einsatz von mehreren Schichten ("Multilayern") besonders sinnvoll, da dadurch eine große Anzahl von Beads zur Erzeugung des Gesamtmagnetfeldes eines Spots beiträgt.

Alternativ oder zusätzlich kann der Aufbau von Molekülnetzwerke zwischen den magnetischen Beads eines Spots unter Verwendung eines chemischen Vernetzers erfolgen. Hier müssen die Fängermoleküle der magnetischen Beads nur an einen Bereich der Zielmoleküle spezifisch binden. Um eine anschließende Vernetzung zu gewährleisten, sind die Zielmoleküle endständig mit einer chemischen Gruppe markiert, die unter bestimmten chemischen oder physikalischen Bedingungen mit gleichartigen chemischen Gruppen eine Vernetzung eingeht. Handelt es sich bei diesem Label z.B. um eine Aminoalkylgruppe, so kann die Vernetzung durch Zugabe von α,ω-Discarbonsäuren wie z.B. α,ω-Polyethylenglykol-Discarbonsäure erfolgen.

Gemäß einer dritten Variante sind nicht nur auf den Beads, sondern auch in der Grundmatrix oder an deren Grenzfläche gegenüber den Zielmolekülen spezifische Fängermoleküle verankert. Im Falle einer Hybridisierung oder Bindung bilden sich Brücken zwischen diesen verankerten Fängern und denen auf der Bead-Oberfläche aus, die eine Drehung des Beads teilweise oder ganz verhindern. Hierbei sind die Fänger auf dem Bead zu einem ersten Bereich und die auf oder in der Matrix verankerten zu einem zweiten Bereich der Zielmoleküle spezifisch. Der Nachweis der Bindungsereignisse erfolgt wie bei der zweiten Variante. Dies gilt wiederum auch für die Verwendung der chemischen Vernetzung. Hier reicht die Adressierung nur einer spezifischen Zielsequenz durch den Fänger, wobei die Zielmoleküle ein vernetzungsfähiges Label tragen müssen.

Zusätzlich zu den Fängermolekülen können die Beads und bei der dritten Variante auch die Grundmatrix mit Streptavidin-Molekülen versehen sein. In diesem Fall werden die Zielmoleküle in einem Bereich am Anfang und, bei der zweiten und dritten Variante, auch am Ende der Zielmolekülkette mit einem Biotin-Molekül verbunden. Im Falle einer Bindung bilden die Zielmoleküle auf diese Weise sehr stabile Molekülbrücken an ein Bead, zwischen den Beads oder zwischen einem Bead und der Grundmatrix, indem sie sowohl über ihren Biotinkomplex also auch über die Fängermoleküle (z.B. mit ihrer spezifischen Sequenz) an den Beads anhaften.

Das beschriebene Verfahren lässt eine besonders hohe Sensitivität erwarten, da bereits wenige Molekülbrücken zu einer Verhinderung der Drehbarkeit der Beads führen sollten. Auf eine Amplifikation der nachzuweisenden Zielmoleküle im Falle von Nukleinsäure-Assays kann daher vorzugsweise verzichtet werden, allerdings ist bei genomischer DNA eine Fragmentierung der DNA häufig zweckmäßig. Der Aufbau der Messvorrichtung wird deshalb durch den Wegfall zahlreicher mikrofluidischer Komponenten stark vereinfacht. Zudem entfällt die bei Amplifikationen übliche Beschränkung auf wenige nachzuweisende Molekülarten (z.B. Multiplex-PCR: Typischerweise < 10 Multiplex-Kanäle).

Um einerseits eine leichte Diffusion der Zielmoleküle zu den Beads und andererseits im Falle einer nachzuweisenden Bindung hohe Haltekräfte der Zielmolekül-Fänger-Komplexe zu erzielen, wird der Vernetzungsgrad der Matrix nach der Hybridisierung in einer bevorzugten Ausführungsform durch eine Nachvernetzung erhöht. Diese Nachvernetzung kann auch zu einer Bindung von Zielmolekül-Fängerkomplexen mit der Matrix über die Verschlingung hinaus führen. In diesem Falle sind die erzielbaren molekularen Haltekräfte besonders hoch. Die Nachvernetzung kann z.B. durch eine Bestrahlung mit UV-Licht, durch Wärme oder durch Zugabe geeigneter Chemikalien erfolgen.

Die Erfindung ist auch auf ein entsprechendes Verfahren zum Nachweis und/oder zur Quantifizierung eines Analyten gerichtet, bei welchem die veränderte Bewegbarkeit eines Nanopartikels in einer Grundmatrix magnetisch ausgelesen wird. Beispielhafte Schreib- und Lesevorgänge werden im Folgenden genauer erläutert.

Die Erfindung wird nun anhand von Ausführungsbeispielen mit Bezug auf die beiliegenden Zeichnungen näher beschrieben. In den Zeichnungen zeigen:
- Fig. 1: einen schematischen Querschnitt durch eine Messvorrichtung gemäß einer ersten Ausführungsform der Erfindung;
- Fig. 2: einen schematischen Querschnitt durch eine Messvorrichtung gemäß einer zweiten Ausführungsform der Erfindung;
- Fig. 3: einen vergrößerten Ausschnitt der Darstellung der Fig. 1, mit an die Beads gebundenen Zielmolekülen gemäß der zweiten Variante;
- Fig. 4: einen vergrößerten Ausschnitt der Darstellung der Fig. 1, mit an die Beads gebundenen Zielmolekülen gemäß einer ersten Ausführungsform der dritten Variante;
- Fig. 5: einen vergrößerten Ausschnitt der Darstellung der Fig. 1, mit an die Beads gebundenen Zielmolekülen gemäß einer zweiten Ausführungsform der dritten Variante;
- Fig. 6: einen vergrößerten Ausschnitt der Darstellung der Fig. 2, mit an die Beads gebundenen Zielmolekülen gemäß der ersten oder dritten Variante;
- Fig. 7: eine vergrößerte Darstellung eines Nanopartikels gemäß Fig. 6 mit einem hybridisierten oder vernetzten Fängermolekül;
- Fig. 8: einen Querschnitt durch einen Spot aus Nanopartikeln, an die keine Zielmoleküle gebunden sind, im Ausgangszustand;
- Fig. 9: einen Querschnitt durch den Spot der Fig. 8 und einen Schreib-Lesekopf beim Schreibvorgang;
- Fig. 10: einen Querschnitt durch den Spot der Fig. 8 und einen Schreib-Lesekopf beim Auslesevorgang;
- Fig. 11: einen Querschnitt durch einen Spot aus Nanopartikeln, an die Zielmoleküle gebunden sind, im Ausgangszustand;
- Fig. 12: einen Querschnitt durch den Spot der Fig. 11 und einen Schreib-Lesekopf beim Schreibvorgang;
- Fig. 13: einen Querschnitt durch den Spot der Fig. 11 und einen Schreib-Lesekopf beim Auslesevorgang.

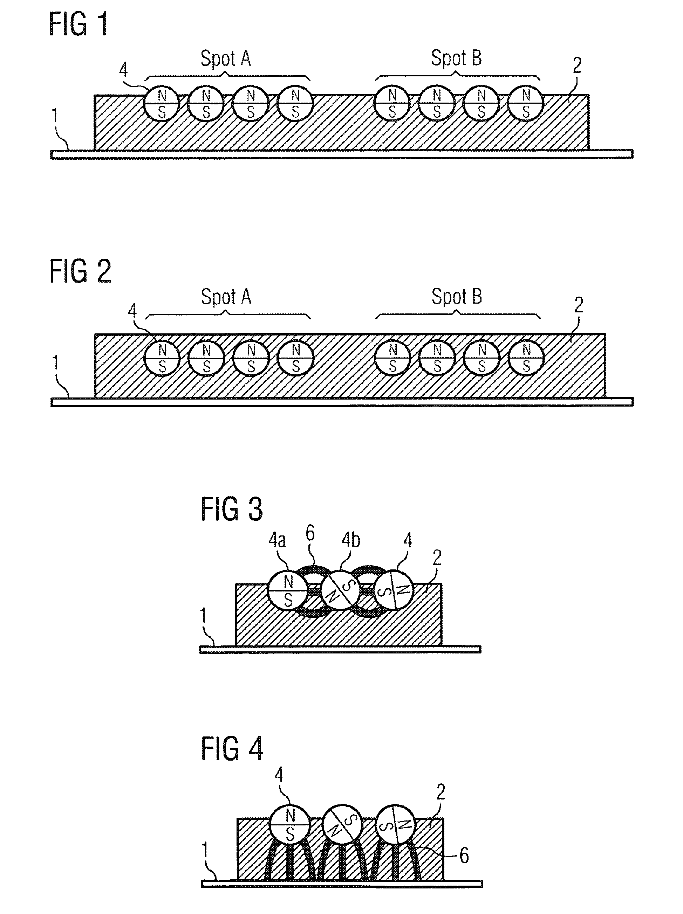

Fig. 1 zeigt schematisch eine Messvorrichtung gemäß einer ersten Ausführungsform der Erfindung. Hier sind sphärische, magnetische Nanopartikel oder Beads 4 an der Oberfläche einer Gelmatrix 2 in diese eingebettet, so dass sie ungefähr bis zur Hälfte vom Gel bedeckt sind. Sie können alternativ auch auf der Oberfläche des Gels aufliegen.

Die Gelmatrix ist auf einem Träger oder einer Basisplatte 1 aus beliebigem Material angeordnet. Wie aus Fig. 1 ersichtlich, sind die Beads 4 zu zwei Spots A und B mit jeweils vier Beads gruppiert. In der Praxis umfasst jeder Spot in der Regel ca. 10-10 Mio., vorzugsweise ca. 1000-1 Mio. Nanopartikel. Diese sind in mehreren Schichten in die Grundmatrix eingebettet.

In der Ausführungsform der Fig. 2 sind die Beads 4 dagegen vollkommen in die Gelmatrix 2 eingebettet. Die auf der Oberfläche der Beads verankerten Fängermoleküle sind in den Fig. 1 bis 6 nicht dargestellt.

Fig. 3 zeigt die Wirkung des Anlagerns von Zielmolekülen bzw. Analyten 6 an die Nanopartikel 4, und zwar bei einer Ausgestaltung der Messvorrichtung gemäß der oben beschriebenen zweiten Variante. Hierbei sind die Beads 4 in einem Spot mit zwei verschiedenen Fängermolekülen ausgestattet, die an unterschiedliche, vorzugsweise weit voneinander entfernte Bereiche der Zielmoleküle binden. Dadurch hybridisiert beispielsweise der Anfang eines gesuchten DNA-Stranges an einen ersten Bead 4a, das Ende des Stranges an einen benachbarten Bead 4b, zur Bildung einer Molekülbrücke 6. Das Bead 4b ist durch z.B. sechs derartiger Molekülbrücken zu z.B. zwei benachbarten Beads 4a und 4 immobilisiert und nicht mehr drehbar.

Bei diesem Ausführungsbeispiel kann entweder jedes Bead in einem Spot mit zwei verschiedenen Fängermolekülen beschichtet sein, oder der Spot enthält eine Mischung aus zwei verschiedenen Arten von Beads mit jeweils einem unterschiedlichen Fängermolekül.

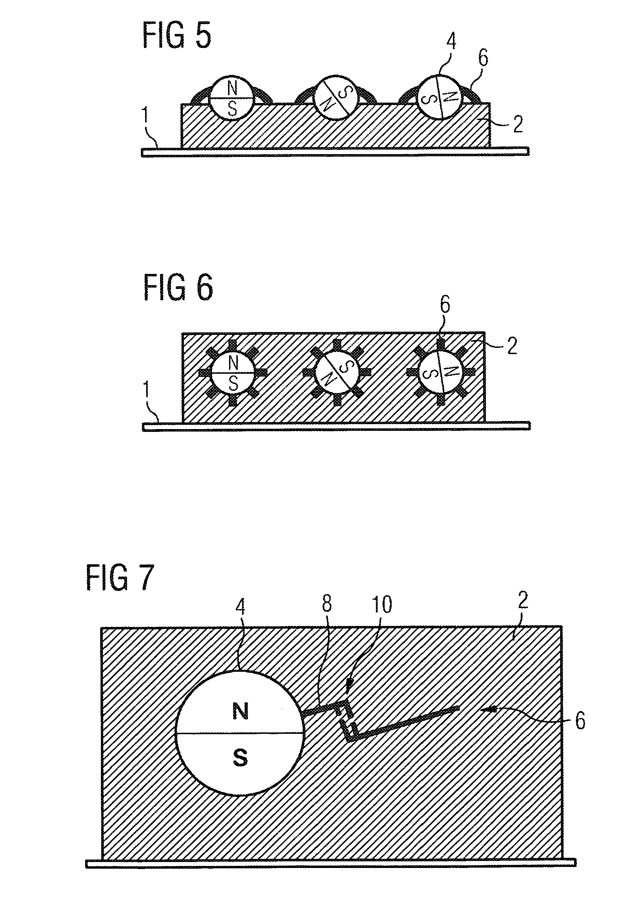

Fig. 4 und 5 zeigen ein Ausführungsbeispiel gemäß der dritten Variante, bei der an der Grundmatrix 2 ebenfalls Fängermoleküle verankert sind. Diese können, wie in Fig. 4 dargestellt, an der Basisplatte bzw. dem Träger 1 verankert sein, oder, wie in Fig. 5 dargestellt, direkt in der Grundmatrix 2 bzw. an deren Grenzfläche zur Umgebung verankert sein. Hier werden durch die Anlagerung von Zielmolekülen an die Fängermoleküle Molekülbrücken 6 zwischen den Beads 4 und der Grundmatrix 2 ausgebildet, durch die die Beads 4 in ihrer Bewegbarkeit und insbesondere Drehbarkeit eingeschränkt werden.

Fig. 6 zeigt ein Ausführungsbeispiel gemäß der oben beschriebenen ersten Variante, bei der einfach durch die Anbindung der Zielmoleküle 6 an die Beads 4 die Bewegbarkeit, insbesondere Drehbarkeit, derselben vermindert wird. Diese Variante empfiehlt sich insbesondere bei großen und/oder sterisch anspruchsvollen Zielmolekülen 6, die nicht ohne Widerstand von den Beads 4 durch die Grundmatrix 2 bewegt werden können. Zusätzlich können die Zielmoleküle 6 gemäß der dritten Variante an ihrem anderen Ende in der Grundmatrix verankert sein.

Fig. 7 zeigt einen Nanopartikel 4 im Gelbett 2 im vergrößerten Querschnitt. Der Nanopartikel wirkt wie ein stabförmiger Permanentmagnet und weist daher einen Nordpol N und einen Südpol S auf. An der Oberfläche des Nanopartikels 4 ist ein Linker 8 verankert, an dem wiederum das Fängermolekül 10 angelagert ist. Das Fängermolekül 10 weist einen Abschnitt auf, der komplementär zu einem Abschnitt eines Zielmoleküls 6 ist und daher an dieses bindet.

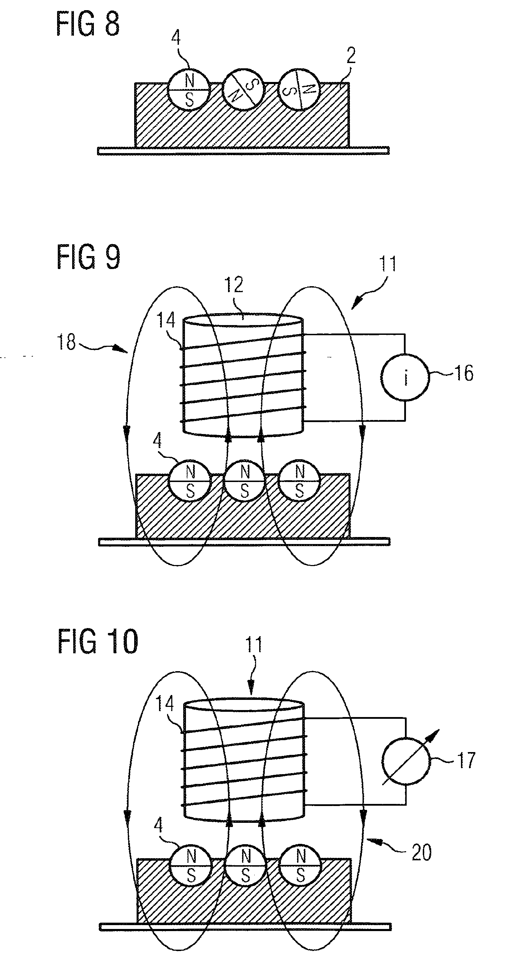

Anhand der Figuren 8 bis 10 und 11 bis 13 soll nunmehr das Nachweisverfahren mithilfe einer magnetischen Auslesung erläutert werden.

In Fig. 8-10 ist ein Messvorgang bei einem Spot aus Beads 4 dargestellt, an den keine Zielmoleküle gebunden sind. Fig. 8 zeigt den Spot im Ausgangszustand. In Fig. 9 ist ein Schreibvorgang dargestellt. Dabei wird durch einen Schreib-Lesekopf 11 ein Magnetfeld 18 in der Grundmatrix erzeugt. Dieses führt dazu, dass sich sämtliche Beads 4 eines Spots in der gleichen Richtung ausrichten, und zwar durch eine Drehung um ihre eigene Achse. In Fig. 9 zeigen die Nordpole N der Beads 4 daher alle nach oben.

Der Schreib-Lesekopf 11 enthält eine beim Schreibvorgang als Elektromagnet wirkende Spule 14 mit einer Wicklung und einem Spulenkern 12. Die Spule 14 ist an eine Stromquelle 16 zur Erzeugung eines über den Spot möglichst homogenen Magnetfelds 18 von definierter Stärke angeschlossen.

Beim Auslesen wird gemäß Fig. 10 mit dem Schreib-Lesekopf 11 das von den ausgerichteten Beads 4 erzeugte Magnetfeld 20 detektiert. Im dargestellten Fall sind die Beads 4 eines Spots mit ihren Nord- und Südpolen alle in einer Richtung ausgerichtet, so dass ein Magnetfeld 20 ausgelesen wird. Dies deutet darauf hin, dass kein Zielmolekül gebunden wurde und dadurch sämtliche Beads des Spots frei drehbar sind. Der Test verlief also negativ.

Der Lese- bzw. Schreibvorgang kann auf verschiedene Weise geschehen:

Zum einen kann beim Schreiben ein statisches Magnetfeld angelegt und beim Auslesen das von den Beads 4 erzeugte statische Magnetfeld gemessen werden, z.B. durch einen im Schreib-Lesekopf 11 enthaltenen (nicht dargestellten) Hall-Sensor oder magnetoresistiven Sensor, z.B. einen GMR, TMR, AMR oder CMR-Sensor.

Zum zweiten kann der Schreib-Lesekopf beim Auslesen die Spots entlang des Arrays abfahren, so dass jeweils bei einer Änderung des Magnetfeldes von einem Spot zum nächsten eine Spannung in der Spule 14 induziert wird. Diese Spannung wird mit einem Spannungsmesser 17 des Schreib-Lesekopfes 11 gemessen. Dieses Vorgehen entspricht dem Prinzip der Auslesung von magnetischen Datenspeichermedien.

Beim ersten und zweiten Verfahren ist es vorteilhaft, jeden Spot zunächst in eine Richtung ("0") zu magnetisieren, das vom Spot erzeugte Magnetfeld auszulesen, und den Spot dann in die entgegen gesetzte Richtung ("1") zu magnetisieren und das Magnetfeld wiederum auszulesen. Dies ist für den Fall wichtig, dass die Beads vor der Bindung mit den Zielmolekülen zufällig gleich ausgerichtet waren, so dass der Spot eine Magnetisierung z.B. in der Richtung "0" zeigt. Dies würde bei einem einmaligen Schreib-Lesevorgang zu dem Schluss führen, dass die Beads sich durch den Schreibvorgang ausgerichtet haben und also ungebunden sind, obwohl sie tatsächlich durch die Bindung mit Zielmolekülen immobilisiert sind.

Zum dritten ist auch eine dynamische Art der Schreibens und Lesens möglich. Hierbei wird die Spule 14 z.B. mit einem Wechselstrom erregt, so dass sie ein periodisch schwingendes magnetisches Feld erzeugt. Die magnetischen Beads 4 fangen daher an, sich mit dem magnetischen Wechselfeld periodisch zu drehen. Durch ihre Trägheit ist diese "Schwingung" ggü. der Schwingung des Magnetfelds leicht verzögert. Bei Abschalten des schwingenden Magnetfeldes schwingen die Beads ebenfalls aufgrund ihrer Trägheit etwas nach. Diese Verzögerung bzw. das Nachschwingen kann anhand der in der Spule 14 induzierten Spannung detektiert werden. Wenn die Beads 4 jedoch an Zielmoleküle 6 gebunden sind, drehen bzw. schwingen sie nicht mit, was ebenfalls detektiert werden kann.

Bei allen Schreib- und Leseverfahren wird der Schreib-Lesekopf 11 zum Schreiben und Lesen eines Arrays aus Spots in einer Messvorrichtung relativ zur Grundmatrix 2, insbesondere entlang der Geloberfläche, bewegt. Dafür kann der Kopf 11 relativ zum Array in der Messvorrichtung, oder die Messvorrichtung relativ zum Kopf bewegt werden. Beides kann in Form einer translatorischen Bewegung (Array aus Spalten und Zeilen) oder einer rotatorischen Bewegung (Kreis- oder Spiralform des Arrays) geschehen.

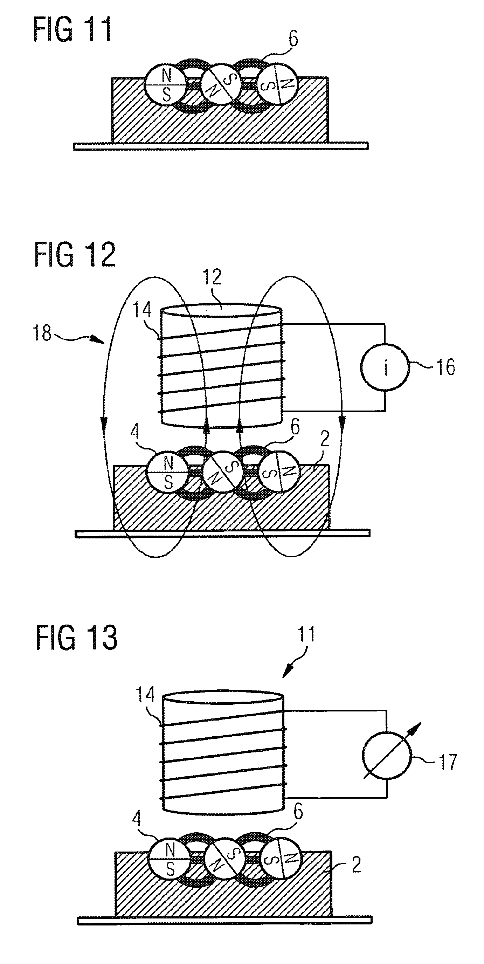

In den Fig. 11 bis 13 ist ein Schreib- und Lesevorgang dargestellt, bei welchem die Anwesenheit von Zielmolekülen dadurch detektiert wird, dass Molekülbrücken 6 zwischen den Beads 4 des Spots gebildet wurden. Der in Fig. 12 dargestellte Schreibvorgang ist daher nicht erfolgreich, die Beads 4 verbleiben in ihrer Ausgangsposition. Dadurch wird durch den Schreib-Lesekopf 11 in dem Spot beim Schreibvorgang keine Magnetisierung erzielt. Die magnetischen Dipole der Beads 4 sind nicht gemeinsam ausgerichtet und erzeugen daher nach außen kein Magnetfeld. Im in Fig. 13 dargestellten Lesevorgang wird daher im Spannungsmesser 17 keine Spannung induziert.

Eine quantitative Messung kann mit dem beschriebenen Verfahren erfolgen, weil das Losreißmoment pro Bead, also dasjenige Drehmoment, das überwunden werden muss, um ein Bead unter Zerreißen der Molekülbrücken oder unter Lösung der Verschlingung mit den Grundmatrixmolekülen zu drehen, von der Anzahl der Molekülbrücken pro Bead und damit von der Konzentration der nachzuweisenden Zielmoleküle abhängt. Das Losreißmoment kann ermittelt werden, indem durch Schreibvorgänge mit steigender Magnetfeldstärke versucht wird, die Magnetisierungsachse der Spots zu drehen. Der Erfolg der jeweiligen Versuche kann durch Lesevorgänge verifiziert werden. Ab einer bestimmten Magnetfeldstärke reißen sich die Beads los bzw. lösen sich und behalten die bei den jeweiligen Schreibvorgängen aufgeprägte Magnetisierungsrichtung.

Alternativ ist eine quantitative Messung dadurch möglich, dass die Anzahl der durch Molekülbrücken fixierten Beads pro Spot von der Konzentration des jeweils nachzuweisenden Probenmoleküls abhängt und den Grad der nach einem Schreibvorgang verbleibenden Magnetisierung 20 (Magnetisierungsremanenz) beeinflusst.

Das Verfahren erlaubt weiterhin zur Optimierung der Genauigkeit vor allem bei quantitativen Messungen eine Kalibrierung der Messvorrichtung. Bei der Herstellung des Arrays kann es zu Spot-zu-Spot-Streuungen der chemischen, mechanischen und/oder magnetischen Eigenschaften kommen, die die Aussagekraft von quantitativen Messungen einschränken. Beispielsweise ist das Gel an verschiedenen Stellen unterschiedlich viskos, so dass die Nanopartikel in verschiedenen Spots eine unterschiedliche Drehbarkeit aufweisen, was wiederum die Ummagnetisierbarkeit jedes Spots beeinflusst. Um diesem Effekt zu begegnen, wird der Grad der Ummagnetisierbarkeit jedes Spots vorzugsweise mit einem oben beschriebenen Schreib-Lesevorgang bereits vor der Zugabe des Probenextrakts, in dem die Zielmoleküle enthalten sind, erfasst. Die nach der Bindung der Zielmoleküle erfassten Messergebnisse lassen sich dann mit den vorher gewonnen Messergebnissen korrigieren.

Bei konventionellen Mikroarrays kommt für SNP- oder Mutationsanalysen häufig eine Variation der Hybridisierungstemperatur zum Einsatz, um die Bindungsfestigkeit der Molekülbrücken zu überprüfen (Temperaturstringenz, Schmelzkurve). Dieses Vorgehen ist bei der hier beschriebenen Anordnung ebenfalls möglich. Es kann dazu z.B. während der Hybridisierung und/oder des Auslesens die Temperatur des Gels und der Beads variiert werden.

Bei einer weiteren Ausführungsform werden die Beads des Arrays bereits bei der Herstellung, also vor der Hybridisierung mit einem Zielmolekül, vormagnetisiert. Dies kann auf eine Weise geschehen, dass Spots oder Teilbereichen von Spots unterschiedliche Magnetisierungsrichtungen eingeprägt werden, also z.B. die in den Figuren dargestellte Magnetisierungsrichtung mit dem Nordpol nach oben, oder eine andere Magnetisierungsrichtung, in der der Nordpol zur Seite zeigt etc.. Die Muster der Magnetisierungsrichtungen können digitale Informationen tragen, die sich nach dem Hybridisierungsvorgang wieder auslesen lassen, sofern am jeweiligen Spot oder Spot-Teilbereich tatsächlich eine Hybridisierung stattgefunden hat (bei Proteinen gilt für Bindungsereignisse sinngemäß das gleiche). Als Informationen, die sich so speichern lassen, kommen z.B. in Frage: Koordinateninformation; dies kann sinnvoll sein, falls sehr einfache, z.B. manuelle Vortriebsmechaniken für den Schreib-Lesekopf eingesetzt werden, oder direkte Aussagen über mit einem Hybridisierungsereignis einhergehende molekularbiologische Erkenntnisse.

Die beschriebenen Ausführungsformen haben u.a. den Vorteil, dass Technologiekomponenten von konventionellen Harddisks übernommen werden können, so z.B. die Bewegungskomponenten der Drehscheibe und des Schreib-Lesekopfs sowie die elektromagnetische Funktion desselben. Die Verwendung neuer magnetische Speichermaterialien und Komponenten aus konventioneller Datenspeichertechnologie erlaubt extrem hohe Informationsdichten, die derzeit im Einsatz befindliche Mikroarray-Systeme übertreffen. Ferner können als Grundmatrix bekannte Kolloide und Gele und bekannte magnetische Partikel verwendet werden. Wegen der zu erwartenden Sensitivität kann für viele Anwendungen auf eine Amplifikation der Zielmoleküle verzichtet werden.

## Patentansprüche

1. Vorrichtung zum Nachweis und/oder zur Quantifizierung eines Analyten (6) mithilfe von Fängermolekülen (10), welche spezifisch an den Analyten binden, welche
- eine Grundmatrix (2) aufweist, und
- mehrere magnetisierte Nanopartikel (4) in der Grundmatrix (2) beweglich angeordnet sind und an denen die Fängermoleküle (10) verankert sind, welche spezifisch an den Analyten (6) binden,
- wobei die Bewegbarkeit der Nanopartikel (4) in der Grundmatrix (2) durch eine Bindung des nachzuweisenden Analyten (6) an die Fängermoleküle (10) beeinflussbar ist und die veränderte Bewegbarkeit der Nanopartikel (4) magnetisch auslesbar ist,
**dadurch gekennzeichnet, dass** die Nanopartikel (4) in mehreren Schichten innerhalb der Grundmatrix (2) angeordnet sind, wobei die Grundmatrix (2) eine Gelmatrix ist.

2. Vorrichtung nach Anspruch 1, **dadurch gekennzeichnet, dass** die Nanopartikel (4) eine zumindest annäherungsweise sphärische oder ellipsoide Form haben.

3. Vorrichtung nach einem der vorhergehenden Ansprüche, **dadurch gekennzeichnet, dass** die Nanopartikel (4) einen Grundkörper aus Kunststoff, insbesondere Polystyrol, aufweisen, der mit magnetischen Partikeln durchsetzt ist.

4. Vorrichtung nach einem der vorhergehenden Ansprüche, **dadurch gekennzeichnet, dass** die Nanopartikel (4) einen Durchmesser von ca. 10nm-10µm, insbesondere von ca. 20nm-1µm und besonders bevorzugt von ca. 50-500nm aufweisen.

5. Vorrichtung nach einem der vorhergehenden Ansprüche, **dadurch gekennzeichnet, dass** die Nanopartikel (4) eine anisotrope Magnetisierung sowie Hysterese-Eigenschaften besitzen.

6. Vorrichtung nach einem der vorhergehenden Ansprüche, **dadurch gekennzeichnet, dass** der nachzuweisende Analyt (6) eine Nukleinsäure oder ein Protein ist und die Fängermoleküle (10) Nukleinsäuren oder monoklonale Antikörper sind, wobei der Analyt (6) durch Hybridisierung bzw. Antigen-Antikörper Reaktion an die Fängermoleküle bindet.

7. Vorrichtung nach einem der vorhergehenden Ansprüche, **dadurch gekennzeichnet, dass** in der Grundmatrix (2) mehrere Nanopartikel (4) zu einem Spot (A, B) räumlich zusammengefasst sind.

8. Vorrichtung nach Anspruch 7, **dadurch gekennzeichnet, dass** in der Grundmatrix (2) mehrere Spots (A, B) von Nanopartikeln (4) vorhanden sind, wobei die Spots (A, B) untereinander jeweils mit verschiedenen Fängermolekülen (10) versehen sind.

9. Vorrichtung nach einem der vorhergehenden Ansprüche, **dadurch gekennzeichnet, dass** die Drehbarkeit der Nanopartikel (4) durch eine Bindung des Analyten (6) an die Fängermoleküle (10) verringerbar ist.

10. Vorrichtung nach einem der vorhergehenden Ansprüche, **dadurch gekennzeichnet, dass** die Nanopartikel (4) in einem Spot (A, B) mit mindestens zwei verschiedenen Fängermolekülen (10) ausgestattet sind, welche an verschiedene Molekülbereiche eines Analyten (6) binden, insbesondere an einen Bereich am Anfang und an einen Bereich am Ende einer Molekülkette des Analyten.

11. Vorrichtung nach einem der Ansprüche 1 bis 10, **dadurch gekennzeichnet, dass** in der Grundmatrix (2) oder an einer Grenzfläche der Grundmatrix (2) ebenfalls Fängermoleküle (10) verankert sind, und zwar verschiedene Fängermoleküle zu denjenigen an den Nanopartikeln (4), wobei die verschiedenen Fängermoleküle (10) an verschiedene Molekülbereiche des Analyten (6) binden, insbesondere an einen Bereich am Anfang und an einen Bereich am Ende einer Molekülkette des Analyten.

12. Vorrichtung nach einem der vorhergehenden Ansprüche, **dadurch gekennzeichnet, dass** der Analyt (6) mit einer chemischen Gruppe gelabelt ist, die unter bestimmten chemischen oder physikalischen Bedingungen mit gleichartigen chemischen Gruppen eine Vernetzung eingeht.

13. Vorrichtung nach einem der vorhergehenden Ansprüche, **dadurch gekennzeichnet, dass** sie ferner einen Schreib-Lesekopf (12) aufweist, mit dem die räumliche Ausrichtung der Nanopartikel (4) oder eines Spots (A, B) von Nanopartikeln (4) durch eine Detektion des von den Nanopartikeln (4) erzeugten magnetischen Feldes (20) gemessen werden kann.

14. Vorrichtung nach einem der vorhergehenden Ansprüche, insbesondere nach Anspruch 7, **dadurch gekennzeichnet, dass** mit dem Schreib-Lesekopf (12) ferner die räumliche Ausrichtung der Nanopartikel (4) oder eines Spots (A, B) von Nanopartikeln (4) durch Anlegen eines bestimmten magnetischen Feldes (18) veränderbar ist, sofern die Beweglichkeit der Nanopartikel (4) nicht durch eine Bindung des nachzuweisenden Analyten (6) an die Fängermoleküle (10) der Nanopartikel (4) beeinflusst ist.

15. Verfahren zum Nachweis und/oder zur Quantifizierung eines Analyten (6) mithilfe von Fängermolekülen (10), welche spezifisch an einen Analyten binden, wobei
- der nachzuweisende Analyt (6) auf eine Grundmatrix (2) aufgetragen wird, in der mehrere magnetisierte Nanopartikel (4) beweglich angeordnet sind, an denen die Fängermoleküle (10) verankert sind, welche spezifisch an einen Analyten (6) binden,
- die Bewegbarkeit der Nanopartikel (4) in der Grundmatrix (2) durch eine Bindung des nachzuweisenden Analyten (6) an die Fängermoleküle (10) beeinflusst wird, und
- eine veränderte Bewegbarkeit der Nanopartikel (4) in der Grundmatrix (2) magnetisch ausgelesen wird,
**dadurch gekennzeichnet, dass** die Nanopartikel (4) in mehreren Schichten innerhalb der Grundmatrix (2) angeordnet sind, wobei die Grundmatrix (2) eine Gelmatrix ist.

16. Verfahren nach Anspruch 15, **dadurch gekennzeichnet, dass** es nach einem der Ansprüche 1 bis 14 ausgestaltet ist.

17. Verfahren nach Anspruch 15 oder 16, **dadurch gekennzeichnet, dass** die veränderte Bewegbarkeit der Nanopartikel (4) durch Anlegen eines Magnetfeldes (18) und anschließendem Auslesen des von mehreren Nanopartikeln (4) erzeugten Magnetfeldes (20) gemessen wird.

18. Verfahren nach Anspruch 17, **dadurch gekennzeichnet, dass** eine Quantifizierung des Analyten (6) durch sequenzielles Anlegen eines Magnetfeldes (18) mit steigender Magnetfeldstärke und jeweils anschließendem Auslesen des von mehreren Nanopartikeln (4) erzeugten Magnetfeldes (20) gemessen wird.

## Claims

1. Device for the detection and/or for the quantification of an analyte (6) with the aid of catcher molecules (10) that bind specifically to the analyte, which
- has a basic matrix (2), and
- a plurality of magnetized nanoparticles (4) are arranged in moveable fashion in the basic matrix (2) and to which the catcher molecules (10) that bind specifically to the analyte (6) are anchored,
- wherein the mobility of the nanoparticles (4) in the basic matrix (2) can be influenced by a binding of the analyte (6) to be detected to the catcher molecules (10) and the changed mobility of the nanoparticles (4) can be read out magnetically, z**characterized in that** the nanoparticles (4) are arranged in a plurality of layers within the basic matrix (2), the basic matrix being a gel matrix (2).

2. Device according to Claim 1, **characterized in that** the nanoparticles (4) have an at least approximately spherical or ellipsoidal form.

3. Device according to one of the preceding claims, **characterized in that** the nanoparticles (4) have a basic body made of plastic, in particular polystyrene, which is permeated with magnetic particles.

4. Device according to one of the preceding claims, **characterized in that** the nanoparticle (4) has a diameter of approximately 10 nm-10 µm, in particular of approximately 20 nm-1 µm, and particularly preferably of approximately 50-500 nm.

5. Device according to one of the preceding claims, **characterized in that** the nanoparticles (4) have an anisotropic magnetization and also hysteresis properties.

6. Device according to one of the preceding claims, **characterized in that** the analyte (6) to be detected is a nucleic acid or a protein and the catcher molecules (10) are nucleic acids or monoclonal antibodies, the analyte (6) binding to the catcher molecules by hybridization or antigen-antibody reaction.

7. Device according to one of the preceding claims, **characterized in that**, in the basic matrix (2), a plurality of nanoparticles (4) are spatially combined to form a spot (A, B).

8. Device according to Claim 7, **characterized in that** a plurality of spots (A, B) of nanoparticles (4) are present in the basic matrix (2), the spots (A, B) in each case being provided with different catcher molecules (10) among one another.

9. Device according to one of the preceding claims, **characterized in that** the rotatability of the nanoparticles (4) can be reduced by a binding of the analyte (6) to the catcher molecules (10).

10. Device according to one of the preceding claims, **characterized in that** the nanoparticles (4) in a spot (A, B) are equipped with at least two different catcher molecules (10) which bind to different molecular regions of an analyte (6), in particular to a region at the start and to a region at the end of a molecular chain of the analyte.

11. Device according to one of Claims 1 to 10, **characterized in that** catcher molecules (10) are likewise anchored in the basic matrix (2) or at an interface of the basic matrix (2), to be precise different catcher molecules than those at the nanoparticles (4), the different catcher molecules (10) binding to different molecular regions of the analyte (6), in particular to a region at the start and to a region at the end of a molecular chain of the analyte.

12. Device according to one of the preceding claims, **characterized in that** the analyte (6) is labeled with a chemical group which undergoes a crosslinking with chemical groups of identical type under specific chemical or physical conditions.

13. Device according to one of the preceding claims, **characterized in that** it furthermore has a write-read head (12), which can be used to measure the spatial orientation of the nanoparticles (4) or of a spot (A, B) of nanoparticles (4) by a detection of the magnetic field (20) generated by the nanoparticles (4).

14. Device according to one of the preceding claims, in particular according to Claim 7, **characterized in that** the write-read head (12) can furthermore be used to change the spatial orientation of the nanoparticles (4) or a spot (A, B) of nanoparticles (4) by application of a specific magnetic field (18) if the mobility of the nanoparticles (4) is not influenced by a binding of the analyte (6) to be detected to the catcher molecules (10) of the nanoparticles (4).

15. Method for the detection and/or for the quantification of an analyte (6) with the aid of catcher molecules (10) that bind specifically to an analyte,
- the analyte (6) to be detected being applied to a basic matrix (2), in which are arranged in moveable fashion a plurality of magnetized nanoparticles (4) to which the catcher molecules (10) that bind specifically to an analyte (6) are anchored,
- the mobility of the nanoparticles (4) in the basic matrix (2) being influenced by a binding of the analyte (6) to be detected to the catcher molecules (10), and
- a changed mobility of the nanoparticles (4) in the basic matrix (2) being read out magnetically,
**characterized in that** the nanoparticles (4) are arranged in a plurality of layers within the basic matrix (2), the basic matrix being a gel matrix (2).

16. Method according to Claim 15, **characterized in that** it is configured according to one of claims 1 to 14.

17. Method according to Claim 15 or 16, **characterized in that** the changed mobility of the nanoparticles (4) is measured by application of a magnetic field (18) and subsequent read-out of the magnetic field (20) generated by a plurality of nanoparticles (4).

18. Method according to Claim 17, **characterized in that** a quantification of the analyte (6) is measured by sequential application of a magnetic field (18) with rising magnetic field strength and in each case subsequent read-out of the magnetic field (20) generated by a plurality of nanoparticles (4).

## Revendications

1. Dispositif de détection et/ou de quantification d'un analyte ( 6 ) à l'aide de molécules ( 10 ) de capture qui se fixent spécifiquement à l'analyte, qui
- a une matrice ( 2 ) de base, et
- plusieurs nanoparticules ( 4 ) aimantées sont disposées de façon mobile dans la matrice ( 2 ) de base et sont ancrées à celles des molécules ( 10 ) de capture qui se fixent spécifiquement à l'analyte ( 6 ),
- dans lequel la mobilité des nanoparticules ( 4 ) dans la matrice ( 2 ) de base peut être influencée par une fixation de l'analyte ( 6 ) à détecter aux molécules ( 4 ) de capture et la modification de mobilité des nanoparticules ( 4 ) peut être lue magnétiquement,
**caractérisé en ce que** les nanoparticules ( 4 ) sont disposées en plusieurs couches au sein de la matrice ( 2 ) de base, la matrice ( 2 ) de base étant une matrice de gel.

2. Dispositif suivant la revendication 1, **caractérisé en ce que** les nanoparticules ( 4 ) ont une forme au moins à peu près sphérique ou en ellipsoïde.

3. Dispositif suivant l'une des revendications précédentes, **caractérisé en ce que** les nanoparticules ( 4 ) ont un corps de base en matière plastique, notamment en polystyrène, qui est traversé par des particules magnétiques.

4. Dispositif suivant l'une des revendications précédentes, **caractérisé en ce que** les nanoparticules ( 4 ) ont un diamètre d'environ 10 nm à 10 µm, notamment d'environ 20 nm à 1 µm, et d'une manière particulièrement préférée d'environ 50 à 500 nm.

5. Dispositif suivant l'une des revendications précédentes, **caractérisé en ce que** les nanoparticules ( 4 ) ont une aimantation anisotrope ainsi que des propriétés d'hystérésis.

6. Dispositif suivant l'une des revendications précédentes, **caractérisé en ce que** l'analyte ( 6 ) à détecter est un acide nucléique ou une protéine et les molécules ( 10 ) de capture sont des acides nucléiques ou des anticorps monoclonaux, l'analyte ( 6 ) se fixant aux molécules de capture par hybridation ou par réaction antigène-anticorps.

7. Dispositif suivant l'une des revendications précédentes, **caractérisé en ce que**, dans la matrice ( 2 ) de base, plusieurs nanoparticules ( 4 ) sont rassemblées dans l'espace en une tache ( A, B ).

8. Dispositif suivant la revendication 7, **caractérisé en ce que**, dans la matrice ( 2 ) de base, sont présentes plusieurs taches ( A, B ) de nanoparticules ( 4 ), les taches ( A, B ) étant munies les unes par rapport aux autres respectivement de molécules ( 10 ) de capture différentes.

9. Dispositif suivant l'une des revendications précédentes, **caractérisé en ce que** l'aptitude à tourner des nanoparticules ( 4 ) peut être diminuée par une fixation de l'analyte ( 6 ) aux molécules ( 10 ) de capture.

10. Dispositif suivant l'une des revendications précédentes, **caractérisé en ce que** les nanoparticules ( 4 ) d'une tache ( A, B ) sont munies d'au moins deux molécules ( 10 ) de capture différentes qui se fixent à des parties de molécule différentes d'un analyte ( 6 ), notamment une partie au début et une partie à la fin d'une chaîne moléculaire de l'analyte.

11. Dispositif suivant l'une des revendications 1 à 10, **caractérisé en ce que**, dans la matrice ( 2 ) de base ou sur une surface d'une limite de la matrice ( 2 ) de base, sont ancrées également des molécules ( 10 ) de capture et, de fait, des molécules de capture différentes de celles sur les nanoparticules ( 4 ), les molécules ( 10 ) de capture différentes se fixant sur des parties de molécule différentes de l'analyte ( 6 ), notamment sur une partie au début et sur une partie à la fin d'une chaîne moléculaire de l'analyte.

12. Dispositif suivant l'une des revendications précédentes, **caractérisé en ce que** l'analyte ( 6 ) est marqué par un groupe chimique qui, dans certaines conditions chimiques ou physiques, subit une réticulation avec des groupes chimiques de même type.

13. Dispositif suivant l'une des revendications précédentes, **caractérisé en ce qu'**il a, en outre, une tête ( 12 ) d'écriture-lecture par laquelle la direction dans l'espace des nanoparticules ( 4 ) ou d'une tache ( A, B ) de nanoparticules ( 4 ) peut être mesurée par une détection du champ ( 20 ) magnétique produit par les nanoparticules ( 4 ).

14. Dispositif suivant l'une des revendications précédentes, notamment suivant la revendication 7, **caractérisé en ce que**, par la tête ( 12 ) d'écriture-lecture, la direction dans l'espace des nanoparticules ( 4 ) ou d'une tache ( A, B ) de nanoparticules ( 4 ) peut être, en outre, modifiée par application d'un champ ( 18 ) magnétique déterminé, dans la mesure où la mobilité des nanoparticules ( 4 ) n'est pas influencée par une fixation de l'analyte ( 6 ) à détecter aux molécules ( 10 ) de capture des nanoparticules ( 4 ).

15. Procédé de détection et/ou de quantification d'un analyte ( 6 ) au moyen de molécules ( 10 ) de capture qui se fixent spécifiquement à un analyte dans lequel :
- on dépose l'analyte ( 6 ) à détecter sur une matrice ( 2 ) de base, dans lequel plusieurs nanoparticules ( 4 ) aimantées sont disposées en étant mobiles, nanoparticules sur lesquelles sont ancrées les molécules ( 10 ) de capture qui se fixent spécifiquement à un analyte ( 6 ),
- la mobilité des nanoparticules ( 4 ) dans la matrice ( 2 ) de base est influencée par une fixation de l'analyte ( 6 ) à détecter aux molécules ( 10 ) de capture, et
- on lit magnétiquement une modification de la mobilité des nanoparticules ( 4 ) dans la matrice ( 2 ) de base,
**caractérisé en ce que** les nanoparticules ( 4 ) sont disposées en plusieurs couches au sein de la matrice ( 2 ) de base, la matrice ( 2 ) de base étant une matrice de gel.

16. Procédé suivant la revendication 15, **caractérisé en ce qu'**il est tel que suivant l'une des revendications 1 à 14.

17. Procédé suivant la revendication 15 ou 16, **caractérisé en ce que** l'on mesure la modification de mobilité des nanoparticules ( 4 ) par application d'un champ ( 18 ) magnétique et en lisant ensuite le champ ( 20 ) magnétique produit par plusieurs nanoparticules ( 4 ).

18. Procédé suivant la revendication 17, **caractérisé en ce que** l'on mesure une quantification de l'analyte ( 6 ) en appliquant séquentiellement un champ ( 18 ) magnétique ayant une intensité de champ magnétique croissante et en lisant ensuite respectivement le champ ( 20 ) magnétique produit par plusieurs nanoparticules ( 4 ).
